# EUROPEAN PATENT APPLICATION

(11) **EP 4 001 424 A1**
(43) Date of publication of application: **25.05.2022**
(21) Application number: 20207541.2
(22) Date of filing: 13.11.2020
(51) Int. Cl.: C12P 33/06, C12N 9/02, C12R 1/01

(54) **SYNTHESIS OF 25-HYDROXY-VITAMIN D3, 25-HYDROXY-7-DEHYDROCHOLESTEROL AND ANALOGOUS ALCOHOLS USING THE BETA-PROTEOBACTERIUM THAUERA AROMATICA**

(71) Applicant: Albert-Ludwigs-Universität Freiburg, 79098 Freiburg (DE)
(72) Inventor: Boll, Matthias, 79194 Gundelfingen (DE); Jacoby, Christian, 79106 Freiburg (DE)
(74) Representative: Krauss, Jan

(57) **Abstract**

The present invention relates to methods using recombinant *Thauera aromatica* cells to produce oxygen-independent molybdenum-hydroxylases in order to convert vitamin D₃, 7-dehydrocholesterol and related alkylated compounds into 25-hydroxy-vitamin D₃, 25-hydroxy-7-dehydrocholesterol and analogous alcohols. The underlying biocatalytic method links the water-dependent hydroxylation of alkyl residues with the nitrate-reducing respiratory chain of the strain as electron acceptor system. In contrast to commonly used methods, the present method does no longer require the artificial regeneration of the electron-carrier.

## Description

The present invention relates to methods using recombinant *Thauera aromatica* cells to produce oxygen-independent molybdenum-hydroxylases in order to convert vitamin D₃, 7-dehydrocholesterol and related alkylated compounds into 25-hydroxy-vitamin D₃, 25-hydroxy-7-dehydrocholesterol and analogous alcohols. The underlying biocatalytic method links the water-dependent hydroxylation of alkyl residues with the nitrate-reducing respiratory chain of the strain as electron acceptor system. In contrast to commonly used methods, the present method does no longer require the artificial regeneration of the electron-carrier.

### Background of the invention

In humans, vitamin D₃ (VitD₃, cholecalciferol) is an essential vitamin that can be formed photochemically in the skin from the precursor 7-dehydrocholesterol (DHC) (Lips 2006). Classically, a deficiency of VitD₃ is associated with osteomalacia and rickets. Today, a series of additional diseases are associated with a vitamin D-deficiency, such as respiratory diseases (including Covid-19), complications during pregnancy, and even cancer and diabetes.

In particular in the northern hemisphere, VitD₃ deficiency is a pandemic. Amongst other factors, this is caused by a reduced exposition to sunlight, impeding the endogenous synthesis of VitD₃ from 7-dehydrocholesterol (DHC) under UV-light (Pilz et al., 2018). In livestock breeding, VitD₃ deficiency is particularly pronounced under artificial light conditions, which negatively influences bone formation and causes substantial impediments of motility.

Vitamin D₃ itself is biologically non-effective, and is metabolized in the liver by cytochrome P450 (CYP) monooxygenases into the circulating storage form 25-OH-VD₃. Thus, the level of serum 25-OH-VitD₃ is an indicator for the VitD-status in the body. Following another hydroxylation in the kidney, the actual and highly effective hormone calcitriol is generated. Supplementation of food by 25-OH-VitD₃ results in much higher 25-OH-VitD₃ serum concentrations than the VitD₃ precursor. For this reason, 25-OH-VitD₃ has to be viewed as a much more effective food supplement for the prevention and treatment of human diseases associated with VitD-deficiency (Cashman et al., 2012). Moreover, the addition of 25-OH-VitD₃ exhibits significant positive effects in farming of pigs and poultry, in particular a strengthening of bone formation and a reduction of diseases caused by VitD₃-deficiency (Wideman et al., 2015, Thayer et al. 2019). The preparation Hy•D^{®} comprising 25-OH-VitD₃ as active ingredient is commercialized by the company DSM.

In analogy to the conversion in the skin, starting from 7-DHC, VitD₃ can be obtained through UV-irradiation in established methods (Escribà-Gelonch et al., 2018). Currently published methods for the conversion of 7-DHC or VitD₃ into the C25-hydroxylated form employ heterologously produced cytochrome p450 (CYP) monooxygenases (as examples: Kang et al. 2015, Abdulmughni et al. 2017, Tang et al. 2020) as bio-catalysts, or chemical synthesis methods (Zhu and Okumara, 1995). In principle, enzymatic methods are superior to chemical methods with respect to selectivity and yield, whereas chemical methods do not involve enzyme catalysts that are only stable within a small range of solvent-, pH, and temperature. Current biocatalytic methods for the synthesis of 25-OH-VitD₃ from VitD₃ by means of microbial CYP monooxygenases depend on the co-substrate O₂ and additional biological electron donor systems comprising NAD(P)H and a NAD(P)H dependent dehydrogenase. Such systems, in turn, require the use of additional enzymatic systems for the regeneration of the electron donor. Systems as described exhibit yield at a maximum of 74.4% (Tang et al. 2020). An essential problem is the lack of selectivity of CYP monooxygenases. In particular the generation of 1,25-dihydroxy-VitD₃, the highly effective hormonal form of VitD₃, is problematic and requires cost-intensive methods for separation.

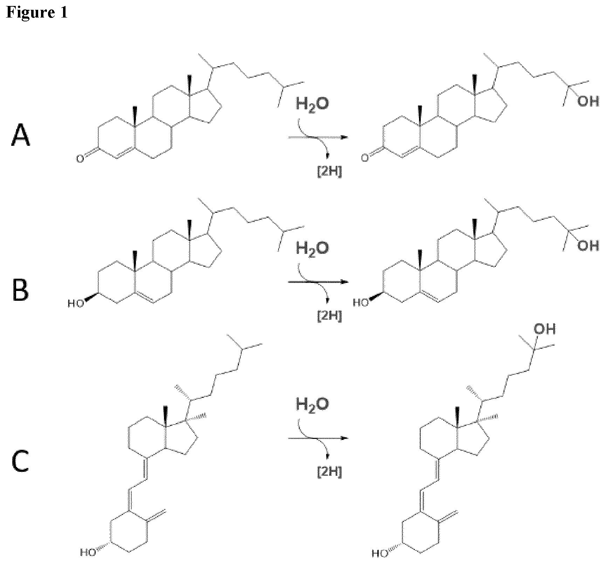

The beta-proteobacterium *Sterolibacterium denitrificans* is able to metabolize steroids as the sole carbon and energy source, such as cholesterol, under denitrifying conditions with nitrate instead of oxygen as terminal electron acceptor of anaerobic respiratory chain. On the basis of polyphasic evidence, the bacterial strain Chol-1S(T) (=DSM 13999T=ATCC BAA-354T) was proposed as the type strain for *Sterolibacterium denitrificans.* During growth with cholesterol, the bacterium produces oxygen-independent, molybdenum-cofactor- containing hydroxylases that use water as hydroxylating agent and an electron acceptor, instead of common CYP monooxygenases. During the decomposition of cholesterol, the enzyme steroid C25 dehydrogenase 1 (S25DH1) catalyzes the C25-hydroxylation of the intermediate cholest-4-ene-3-one (Figure 1A).

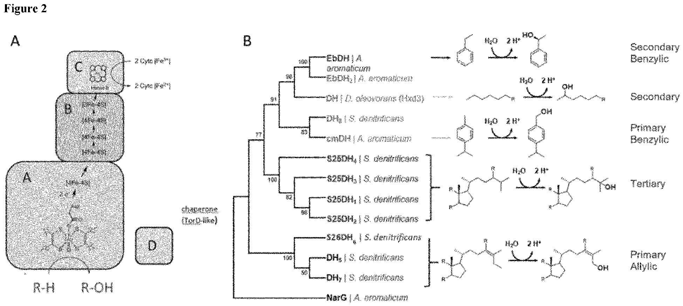

The prototypic enzyme S25DH1 was isolated and characterized from the wildtype strain of *S. denitrificans* (Dermer et al., 2012). The catalytic reaction takes place at a molybdenum-cofactor bound to the A-subunit of the enzyme. The B- and C- subunits provide an electron transfer chain to the natural acceptor cytochrome c. A chaperone, also imprecisely designated as D-subunit, is important for the folding of the trimeric enzyme complex (Fig. 2A). S25DH1 belongs to a class of related molybdoenzymes having the same structure and cofactor equipment, that all hydroxylate C-H-residues in tertiary, secondary or primary-allylic or primary-benzylic position using water as hydroxyl donor (Fig. 2B). S25DH1 hydroxylates the tertiary C25 of the side chain of cholest-4-ene-3-one with a high specificity (>99%), and only requires a simple one-electron acceptor, such as non-biologic Fe³⁺ (for example K₃Fe[CN]₆). Nevertheless, for continuous reactions and/or operation, this acceptor has to be regenerated.

PL226816B1 discloses the use of the enzyme C25DH1 from *Sterolibacterium denitrificans* wildtype either in purified form or present in a cellular extract only for the conversion of DHC into 25-OH-DHC.

*Thauera aromatica* K172 (DSM 6984) is a facultatively anaerobic, mesophilic beta-proteobacterium that was isolated from sewage sludge (Anders HJ, et al. 1995). When grown with organic compounds such as benzoate, it can use nitrate as electron acceptor in an anaerobic respiratory chain, thereby employing a molybdenum-cofactor containing nitrate reductase related to S25DHs.

Due to the above-mentioned limitations of enzymatic and chemical procedures there is still a need in the art for new approaches to convert of VitD₃, DHC and analogous alkyl-substrates into 25-OH-VitD₃, 25-OH-DHC or analogous hydroxylated products. It is therefore an object of the present invention to provide new improved methods and cells that can be efficiently used in novel approaches. Other problems and advantages of the present invention can be readily derived by the person of skill when studying the following description.

In a first aspect thereof, the present invention solves the above problem by providing a method for producing 25-hydroxy-vitamin D₃, 25-hydroxy-7-dehydrocholesterol (25-OH-7-DHC) and/or analogous alcohols thereof, comprising a) providing a recombinant strain of a suitable beta-proteobacterium, preferably *Thauera aromatica* K172, that heterologously overexpresses the genes encoding the three structural genes of at least one functional water-dependent alkyl-hydroxylase together with at least one chaperone cofactor, b) suitably culturing said strain in or on a culture medium with nitrate as terminal electron acceptor for anaerobic respiration in order to obtain resting cells of said strain, c) adding vitamin D3 (VitD3) and/or 7-dehydrocholesterol (7-DHC) or other suitable compound comprising a C-H-residue in a tertiary, secondary or primary-allylic or primary-benzylic position to be hydroxylated to said resting cells, and, optionally, d) isolating said 25-OH-VitD₃, 25-OH-7-DHC and/or said analogous alcohol thereof from said culture medium.

Preferably, beta-proteobacterium is *Thauera aromatica* K172 heterologously overexpressing the genes encoding *S. denitrificans* S25DH1, *S. denitrificans* S25DH2, *S. denitrificans* S25DH4 and/or *S. denitrificans* S25DH2-4, together with the chaperone as required.

Advantageously, the method according to the present invention does not produce calcitriol and/or does not produce substantial amounts of calcitriol or other undesired by-products (see also below).

The method according to the present invention provides high yields with a conversion of >75%, preferably >80%, more preferably >90%, and most preferably >95%, such as >99%.

More preferably, the method according to the present invention is performed as a continuous method, or a two-phase method.

In a second aspect thereof, the present invention solves the above problem by providing a method according to the present invention, wherein said method is performed with the recombinant strain in the form of a biofilm, preferably a biofilm on an electrode material.

In a third aspect thereof, the present invention solves the above problem by providing the use of a recombinant strain of a beta-proteobacterium, preferably *Thauera aromatica* K172, that heterologously overexpresses the genes encoding the structural subunits of at least one functional water-dependent alkyl-hydroxylase together with at least one chaperone cofactor for the production of 25-hydroxy-vitamin D₃ (25-OH-VitD3), 25-hydroxy-7-dehydrocholesterol (25-OH-7-DHC) and/or analogous alcohols thereof, preferably in a method according to the present invention.

It was found by the present inventors in 2016 that cellular extracts of *S. denitrificans,* when grown on cholesterol, in addition to the natural substrate cholest-4-ene-3-one, in a highly specific and complete (>99%) fashion convert both DHC as well as VitD₃ into the 25-OH-products (Figure 1), in the same manner as the enzyme S25DH1 when purified from cell extracts. The conversion of VitD₃ additionally requires at least 5% 2-hydroxypropyl-□-cyclodextrin, in order to move the equilibrium into the direction of pre-VitD₃, the actual substrate of S25DH1 (Warnke et al., 2016).

Jacoby et al. (2018) established a heterologous expression platform for the three structural genes of S25DH subunits together with an essential chaperone in the denitrifying beta-proteobacterium *Thauera aromatica* K172. Using this system, S25DH1 and three isoenzymes (S25DH2, S25DH3, and S25DH4) were overproduced in a soluble, active form allowing a straightforward purification of non-tagged αβγ complexes. All S25DHs catalyzed the specific, water-dependent C-25 hydroxylations of various 4-en-3-one forms of phytosterols and zoosterols. Crude extracts from *T*. *aromatica* expressing genes encoding S25DH1 catalyzed the hydroxylation of vitamin D3 (VD3) to the clinically relevant 25-OH-VD3 with >95% yield at a rate 6.5-fold higher than that of wild-type bacterial extracts; the specific activity of recombinant S25DH1 was found to be twofold higher than that of the wild-type enzyme.

In contrast to Jacoby et al. (2018), the present invention uses whole intact cells of *Thauera aromatica* K172 that heterologously overexpress S25DH1, S25DH2 or related water-dependent alkyl-hydroxylases. In the present case, the genes encoding the three subunits of S25DH1 and S25DH2 and the chaperone are expressed from a recombinant plasmid. Under denitrifying conditions this allows for a selective conversion of VitD₃, DHC and analogous alkyl-substrates into 25-OH-VitD₃, 25-OH-DHC or analogous hydroxylated products.

The methods as disclosed involving S25DH-variants for the enzymatic hydroxylation of VitD₃ or DHC at position C25 require a cost-intensive production of extracts from cells heterologously producing the enzyme. The use of the wild type *Sterolibacterium denitrificans* as described in Warnke et al., 2016 and PL226816B1 further requires the use of inhibitors in order to avoid side reactions. Even more problematic is the time-consuming, ineffective and cost-intensive purification of the enzymes using chromatographic methods, in order to avoid side reactions. In addition, these methods have the disadvantage that enzymes are generally more unstable outside of cells than in the cellular environment (under aerobic conditions, the stability of the activity of extracts comprising S25DH1 drops for <10% after 24 h, the purified enzyme is even less stable). Finally, cell free methods require the *in vitro* regeneration of the electron acceptor either through the use of additional enzyme systems or the use of electro-catalytic methods.

A couple of advantages were surprisingly identified in the context of the present invention that were neither disclosed nor proposed in Jacoby et al. (Jacoby et al 2018).
i) The bacterium produces the enzymes in the periplasm. This is a prerequisite for the conversion of Vitamin D3 without uptake thereof into the cells.
ii) When using whole cells of T. aromatica under conditions nearly identical to those in the wild-type, the stability of the enzyme is much higher than in artificially prepared cell extracts.
iii) The process makes use of nitrate as convenient and cost-efficient electron acceptor using the intrinsic respiratory chain of the denitrifying *T*. *aromatica.* Nitrate is converted into the unproblematic dinitrogen gas, per Vitamin D3 only 0.4 equivalents of nitrate are used, and not two equivalents ferricyanide (Jacoby et al., 2018). Therefore, less electron acceptor is required, and nitrate can be continuously supplied, preferentially by continuous feeding in order to avoid nitrate accumulation.
iv) The present method does not produce essential amounts of the difficult and expensive to be removed byproduct calcitriol or other contaminants.
v) For the industrial production, *Thauera aromatica* K172 can be grown to a high density of cells, and resting cells can be used in order to produce the desired products with high yield.

The present method and the respective organism are also substantially different from, for example, PL226816B1 in that the methods as disclosed in PL226816B1 exclusively relate to S25DH1, in contrast, the use of other molybdenum-containing alkylhydroxylases, such as, for example, S25DH2 is neither disclosed nor proposed. S25DH2 exhibits a significantly higher activity with DHC and is 44-fold higher in the present strain *T*. *aromatica* K172 when compared to the wild type.

Furthermore, all methods as disclosed in PL226816B1 relate to the conversion of cholest-4-ene-3-one, cholest-1,4-diene-3-one, cholest-4,6-diene-3-one, DHC as well as cholesterol-succinate or hydroxymethylglycinate by means of C25DH1. No biocatalytic conversion of VitD₃ is described or contemplated.

All methods as disclosed in PL226816B1 disclose the use of S25DH1 from the wild type-strain *Sterolibacterium denitrificans* Chol1 in isolated form or in cell extracts, but no whole cell systems are disclosed.

All methods as disclosed in PL226816B1 relate to the use of S25DH1 from the wild type-strain *Sterolibacterium denitrificans* Chol1, but not to the heterologous production of the genes in the production strain *Thauera denitrificans K172.*

The method according to the present invention is performed with a suitable beta-proteobacterium, preferably *Thauera aromatica* K172. Further preferred are bacteria selected from the genus *Rhodocyclales,* for example *Sterolibacterium denitrificans.* Bacteria are selected based on their suitability to be grown in culture, and their safety levels, e.g. level S1.

In general, any recombinant water-dependent, molybdenum-cofactor-containing alkyl-hydroxylase can be used in the methods according to the present invention, as long as it hydroxylates, and preferably selectively hydroxylates, a C-H-residue in a tertiary, secondary or primary-allylic or primary-benzylic position of a compound, such as vitamin D3 (VitD3) and/or 7-dehydrocholesterol (7-DHC). Preferred enzymes as used in the context of the present invention are shown in Figure 2B. The water-dependent alkyl-hydroxylase is preferably selected from an enzyme of the group steroid C25 dehydrogenase 1 (S25DH1) enzymes and related molybdoenzymes and related water-dependent alkyl-hydroxylases. All these enzymes consist of three subunits, wherein the A-subunit comprises the active site and determines both reactivity and selectivity. The additional B- and C-subunits can be exchanged, as can be the chaperone as described below. Thus, related enzymes in the context of the present invention are enzymes, such as bacterial enzymes, that are defined by a functional active site, molybdenum-cofactor-harboring A-subunit of the dimethyl sulfoxide reductases (DMSO) family of molybdenum-cofactor-containing hydroxylases, preferably with an amino acid sequence identity of the A-subunit of 35% or more, preferably of 50% or more, compared to the A-subunit of the enzyme S25DH1, enzymes that have the same structure (e.g. are molybdoenzymes or have the same subunit structure) and/or perform the same function, i.e. are able to hydroxylate, and preferably selectively hydroxylate, a C-H-residue in a tertiary, secondary or primary-allylic or primary-benzylic position of a compound, such as vitamin D3 (VitD3) and/or 7-dehydrocholesterol (7-DHC), more preferably under the conditions as described herein (see also the alkyl hydroxylases as listed in Fig. 2B). Related enzymes also include enzymes sharing an amino acid identity of more than 80%, preferably more than 90%, and even more preferred of more than 95% with the subunits of *S. denitrificans* S25DH1 and/or the subunits of *S. denitrificans* S25DH2. Preferred are enzymes selected from *S. denitrificans* S25DH1, *S. denitrificans* S25DH2, *S. denitrificans* S25DH4, and *S. denitrificans* S25DH2-4 or related enzymes thereof as disclosed herein. Particularly preferred are enzymes selected from *S. denitrificans* S25DH1, *S. denitrificans* S25DH2, *S. denitrificans* S25DH4, and *S. denitrificans* S25DH2-4 that are mutated in order to achieve a higher hydroxylation rate of vitamin D3 (VitD3) and/or 7-dehydrocholesterol (7-DHC) as disclosed herein, in particular in the context of the production strain *Thauera denitrificans K172.*

In the context of the method according to the present invention, at least the B- and C-subunits and at least one chaperone cofactor have to be co-expressed in the recombinant production strain, in order to provide functional and properly folded water-dependent alkyl-hydroxylases. Preferably, said at least the B- and C-subunits one and at least one chaperone cofactor to be expressed is selected from the respective BCD-subunits thereof and related cofactors thereof, such as *S. denitrificans* S25-DH1 D-subunit. Expression can be achieved using the same plasmid as for the expression of the water-dependent alkyl-hydroxylase(s), and/or a separate one. Respective plasmids are disclosed in the literature, and known by the person of skill in the art.

Preferred is the method according to the present invention, wherein said at least one water-dependent alkyl-hydroxylase subunit and at least one chaperone cofactor are expressed from a recombinant plasmid, preferably further comprising a selection marker, such as gentamycin resistance. A further preferred example is described in the example, below.

Other options for producing the enzymes and/or parts thereof as used in the context of the present invention comprise expression cassettes that are integrated into the bacterial genome. Respective elements are disclosed in the literature, and known by the person of skill in the art.

The heterologous expression or overexpression can be controlled, preferably using a suitable induction (chemically (e.g. IPTG), temperature, co-factors, genetic elements for controlling expression) of the recombinant enzymes.

Advantageously, the method according to the present invention uses water as hydroxylating agent for the conversion(s) as described herein.

In the method according to the present invention, the recombinant production strain is suitably (i.e. suitable medium, temperature, pressure, vessel(s), pH) cultured in (e.g. liquid) or on (e.g. plate or surface) a culture medium with nitrate as terminal electron acceptor for anaerobic respiration in order to obtain resting cells of said strain, before the addition of vitamin D3 (VitD3) and/or 7-dehydrocholesterol (7-DHC) or other suitable compound to be converted. Preferably, in the method according to the present invention, culturing is performed in a medium, preferably a synthetic minimal medium, comprising benzoate, ethanol or acetate as electron donor and carbon-source and nitrate as electron acceptor under anaerobic conditions.

Furthermore, it was surprisingly found that for the method according to the present invention the culturing can take place at a high density of cells and/or with resting (i.e. non-growing or slowly growing) cells. This is advantageous for industrial methods for the conversion as disclosed herein. Preferably, cells in the late exponential phase (OD₅₇₈ₙₘ ≥ 5) are used, which can be adjusted to densities of between 50-150 (OD₅₇₈ₙₘ).

More preferably, culturing takes place at about 30°C, or at between 28-37°C.

It is a particularly advantageous feature of the method according to the present invention that it does not produce calcitriol and/or does not produce substantial amounts of calcitriol or other undesired by-products. In particular in view of the biologic effects of this highly effective hormone, calcitriol is undesired in the method, and needs to be removed, which is difficult and cost-intensive.

In another aspect of the method according to the present invention, said method can further comprise the addition of an electron acceptor, such as Fe³⁺, for example K₃Fe[CN]₆, DMSO, N₂O, O₂ or other inorganic electron acceptor. Respective acceptors are disclosed in the literature, and known by the person of skill in the art. Nevertheless, the system of the nitrate-reducing respiratory chain of the producing strain as electron acceptor system is preferred.

It is a particularly advantageous feature of the method according to the present invention that said method provides a yield of the conversion of >75%, preferably >80%, more preferably >90%, and mot preferably >95%, such as >99% (see also table 2, below). These yields are not reached by other whole cell systems as known.

Preferred is a method according to the present invention, wherein said method is performed as a continuous method or a two-phase method, such as liquid-liquid or liquid-solid. Further preferred is a method according to the present invention, wherein said method is performed as a (static), fed-batch' method. This is advantageous for industrial methods for the conversion as disclosed herein. The converted 25-OH-VitD₃, 25-OH-7-DHC and/or the analogous alcohols thereof can be conveniently isolated from the medium, and as a consequence the whole cells can be used for an extended period of time.

Preferred is a method according to the present invention, wherein said method further comprises the addition of a suitable amount of cyclodextrine, such as 2-hydroxypropyl)-beta-cyclodextrine (HPCD), for example as disclosed in the examples, below.

In another aspect of the method according to the present invention, the method is performed with the recombinant strain of a suitable beta-proteobacterium, preferably *Thauera aromatica* K172, as a biofilm, preferably a biofilm on an electrode material.

Yet another aspect of the present invention then relates to the use of a recombinant strain of a beta-proteobacterium, preferably *Thauera aromatica* K172, that heterologously overexpresses at least one functional water-dependent alkyl-hydroxylase composed of the ABC-subunits and at least one chaperone cofactor for the production of 25-hydroxy-vitamin D₃ (25-OH-VitD3), 25-hydroxy-7-dehydrocholesterol (25-OH-7-DHC) and/or analogous alcohols thereof as disclosed herein. Preferred is the use in a method according to the present invention.

The invention relates to a synthesis of 25-hydroxy-vitamin D₃ (25-OH-VitD3), 25-hydroxy-7-dehydrocholesterol (25-OH-7-DHC) and/or analogous alcohols from alkyl-precursors thereof as disclosed herein using whole cells, such as the preferred recombinant *Thauera aromatica* K172, heterologously expressing plasmid-encoded genes or genes that were artificially introduced into the genome of S25DH-variants and related water-dependent alkyl hydroxylases that serve for the selective hydroxylation of steroid-side chains as well as of other alkyl groups in tertiary, secondary, primary-benzylic or primary-allylic positions (see the alkyl hydroxylases as listed in Fig. 2B).

The invention also relates to methods, wherein the alkyl hydroxylases transfer electrons to alternative biological electron acceptor systems, such as respiratory chains with nitrate, alternatively with DMSO, nitrite, N₂O, O₂ or other inorganic electron acceptors, as well as artificial electron acceptor systems with Fe(CN)₆³⁺ or analogous artificial one-electron-carriers that transfer the electrons from the *T*. *aromatica* K172 cells to an anode. This includes biofilms of recombinant *T*. *aromatica* K172 on electrode materials.

The invention shall now be further described in the following examples with reference to the accompanying Figures, nevertheless, without being limited thereto. For the purposes of the present invention, all references as cited herein are incorporated by reference in their entireties.
Figure 1 shows a diagram of the water-dependent specific C25-hydroxylations as catalyzed by S25DH1. Hydroxylations of A, the natural substrate cholest-4-ene-3-one, B, 7-dehydrocholesterol, C, Vitamin D₃.
Figure 2 shows the general subunit-architecture and cofactor composition of enzymes belonging to the class of molybdoenzymes that catalyze an oxygen-independent hydroxylation of alkyl groups. Figure 2B shows phylogenetically related, molybdenum-containing A-subunits of oxygen-independent hydroxylases as used in accordance with the present invention and their reactions as catalyzed.

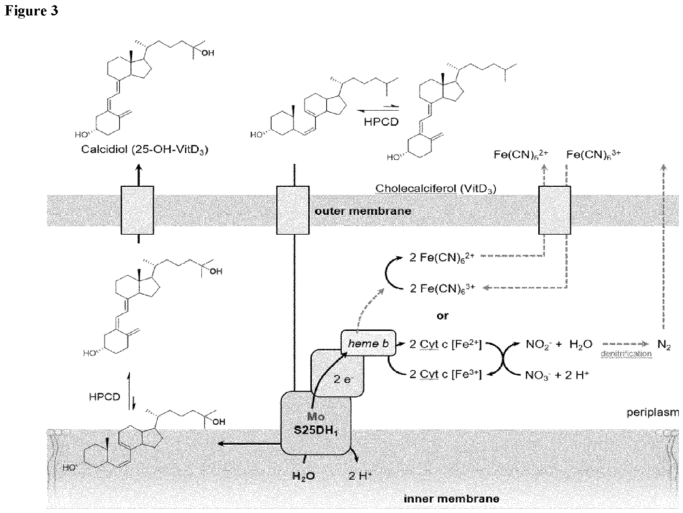

Figure 3 schematically shows the use of intact *Thauera aromatica* K172 cells that heterologously overexproduce the three subunits of S25DH1 for the selective hydroxylation of Vitamin D₃ into 25-OH-VitD₃. Enzymes of the respiratory nitrate oxidation are electronically coupled to the S25DH1 by endogenous cytochrome c. Variants: (i) analogously, 7-DHC is hydroxylated by S25DH1 into 25-OH-7-DHC; (ii) alternatively, alkyl group-hydroxylating enzymes that are similar to S25DH1 are heterologously produced in *T*. *aromatica* for a synthesis using whole cells (for this, see Fig. 2); (iii) as an alternative to nitrate or other terminal acceptors of endogenously present respiratory chains Fe(CN)₆³⁺ may be used as (additional or alternative) artificial acceptor, which could be regenerated through the interaction of whole cells with an anode, for example in form of a biofilm.
Figure 4 shows the analysis of the efficiency (yield) of the conversion of (A) VitD₃ into 25-OH-VitD₃, and (B) 7-DHC into 25-OH-7-DHC by whole cells of *T*. *aromatica* K172 heterologously producing (A) S25DH1, and (B) S25DH2, respectively, according to the present invention.
Figure 5 shows the plasmid map of vector pIZ1016 as used for the production of the alkyl-hydroxylases in *Thauera aromatica* K172. The tac promoter was used for the production of the enzymes.

### Examples

### Methods

*Thauera aromatica* K172 is grown under denitrifying conditions in a sealed and airtight vessel (2 L, N₂-atmosphere) in mineral salt medium (0.6 g L⁻¹ NaH₂PO₄ x 1 H₂O, 7.3 g L⁻¹ K₂HPO₄ x 3 H₂O, 0.54 g L⁻¹ NH₄Cl). Trace elements (1.5 g L⁻¹ FeCl₂ x 4 H₂O, 70 mg L⁻¹ ZnCl₂, 100 mg L⁻¹ MnCl₂ x 4 H₂O, 2 mg L⁻¹ CuC12 x 2 H₂O, 190 mg L⁻¹ CoCl₂ x 6 H₂O, 24 mg L⁻¹ NiCl₂ x 6 H₂O, 36 mg L⁻¹ Na₂MoO₄ x 2 H₂O, 6 mg L⁻¹ H₃BO₃ dissolved in 12.8 g L⁻¹ and 5.6 g L⁻¹ NaOH), vitamins (200 mg L⁻¹ nicotinic acid, 20 mg L⁻¹ D(+)-Biotin, 20 mg L⁻¹ thiamine dichloride, 80 mg L⁻¹ 4-aminobenzoic acid, 100 mg L⁻¹ Ca-D(+)-pantothenic acid, 300 mg L⁻¹ pyridoxine-dihydrochloride, 100 mg L⁻¹ cyanocobalamine), 1 M MgSO₄ and 0.1 M CaCl₂ were each added in a 1:1000 dilution. In addition, 5 mM sodium benzoate (carbon source) and 15 mM sodium nitrate (ratio 1:3) were added. Culturing was done at 30 °C. After consumption of benzoate/nitrate, sodium benzoate and sodium nitrate in a ratio of 1:3 were added using a pump in a fed-batch fashion. No formation of undesired nitrite was found under these nitrate-limited conditions.

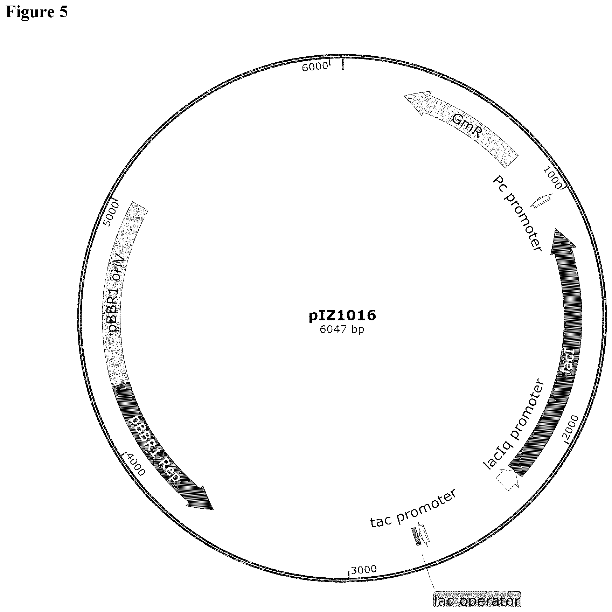

The genes of the alkyl-hydroxylases as used in the present invention were cloned into the broad-host-range vector pIZ1016 (Gm^{r}, *ori*pBBR1, Mob⁺, *lacZα, Ptac*/*lacI^{q}*) (Fig. 5) and transformed into *Thauera aromatica* K172 as described (Jacoby et al., 2018).

The expression of the genes took place in the early exponential growth phase in mineral salt medium (20 µg mL⁻¹ gentamicin sulfate) at an optical density (OD₅₇₈ₙₘ) of 0.6 - 0.8 and induction using 1 mM isopropyl-β-D-thiogalactopyranoside (IPTG). The cells were induced for 72 - 96 hours (OD₅₇₈ₙₘ ≥ 5) through the continuous addition of sodium benzoate and sodium nitrate (1:3) before they were used for the conversion of VitD3, DHC or others as hydroxylating alkyl-compounds in cell suspension experiments.

For the conversion, cells in the late exponential phase (OD₅₇₈ₙₘ ≥ 5) were used, cooled down (<10 °C) and centrifuged (6000 rpm, 4 °C, 10 min). Then, the cells were washed 2x in 50 mM MOPS/KOH buffer pH 7.0 in order to remove excess benzoate, nitrate and opt. lysed cells. Following suspension in the respective volumes of 50 mM MOPS/KOH buffer pH 7.0, an OD₅₇₈ₙₘ was adjusted to be at a value of between 10-150. The conversion of vitamin D₃ took place in a closed vessel as follows: 7.5% (w/v) (2-hydroxypropyl)-beta-cyclodextrine (HPCD), 2,5 mM vitamin D₃ and 10 mM sodium nitrate (alternatively: 10 mM K₃[Fe(CN)₆]) were added to the preparation. The transformation of alkyl-substrates into hydroxylated products happened in a time-dependent manner at a temperature of 30 °C and in case of the conversion of VitD₃ or DHC is detected by means of ultra-performance-liquid chromatography (UPLC) (Jacoby et al., 2018).

As also described above, the use of wild type-S25DH1 from *S. denitrificans* for a conversion of VitD₃ and DCH into the C25-hydroxylated products comes with cost-intensive disadvantages. When using cellular extracts of *S. denitrificans,* the enzymes for cholesterol degradation as produced by the wild type convert 25-OH-VitD₃/25-OH-7-DHC into by-products, a phenomenon that can only be avoided using the harmful and expensive inhibitor AgNO₃ (Warnke et al., 2016).

The alternative, time-consuming and cost-producing isolation of the enzyme in order to avoid these side reactions, in turn, causes an essential loss of activity.

In order to overcome the above disadvantages, the three structural genes of S25-DH1 (Genbank Accession-No. SDENCHOL_20804, SDENCHOL_20461, and SDENCHOL_20462) as well as the gene of a chaperon (SDENCHOL_20207) were cloned into a plasmid comprising a gentamycin-resistance-cassette, and transformed into the recombinant host strain *Thauera aromatica* K172 (beta-proteobacterium, safety category S1). The resulting strain was grown with benzoate as electron donor and carbon-source and nitrate as electron acceptor under anaerobic conditions. In cellular extracts as produced by French Press, the S25-DH1-activity was found to be already 6.4-fold higher than in the *S. denitrificans* wild type (Jacoby et al., 2018) (Table 1), a result that was also obtained using the homologous enzyme S25DH2-4, an enzyme that convert different steroids with different activities (Jacoby et al., 2018) (Fig. 2B).

Amongst the S25DH-variants, S25DH2 was found to be the most promising enzyme with respect to the conversion of DHC into 25-OH-DHC. In this case a 44-fold increase was found in cell extracts compared with the *Sterolibacterium denitrificans* wild type (Jacoby et al. 2018) (Table 1). These activities can both be detected in extracts of *T*. *aromatica* K172 and with the purified enzymes. For a stable production, gentamycin was added to the producing cells as selection agent.

**Table 1: Comparison of the activities of wild type-S25DH1 and of the heterologously produced S25D1, S25DH2 from cells of T. aromatica K172 with different steroid-substrates in cellular extracts.**

| **Enzyme** | **Cholest-4-ene-3-one** | **DHC** | **VitD₃** |
|---|---|---|---|
| Wild type S25DH1 | 100% | 13% | 24% |
| Recombinant S25DH1 | 642% | 82% | 154% |
| Recombinant S25DH2 | 497% | 563% | 32% |

*T*. *aromatica* was grown in a ,fed-batch' method with benzoate and nitrate under anaerobic conditions to an optical density of 5-6, wherein a nitrate/benzoate-mixture in a ratio of 3:1 was continuously added to the culture. Consequently, the cells grow under nitrate-limitation, hence avoiding an undesired nitrite formation. Subsequently using centrifugation and resuspension in a medium without nitrate and benzoate the cells were adjusted to an optical density of between 10-100.

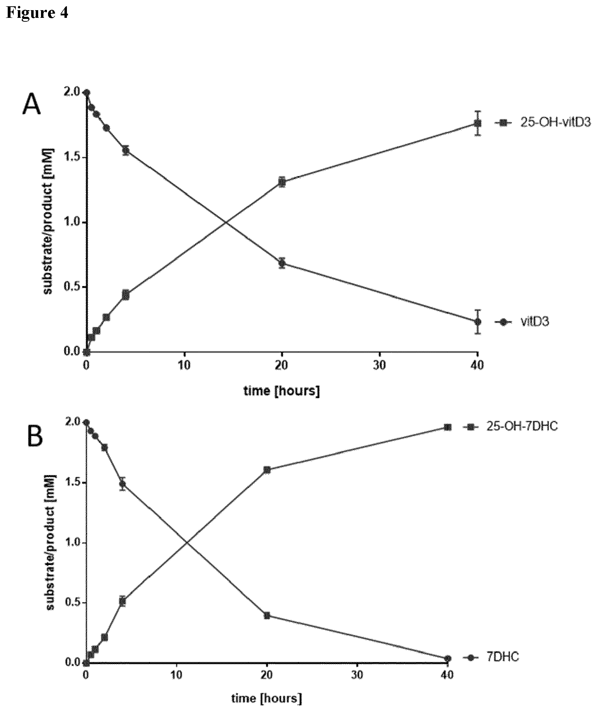

It was surprisingly found that in *Thauera aromatica* K172 ,resting-cells' that were generated as described above and heterologously produced the three genes of S25DH-variants and the chaperone as required for proper folding the following processes took place that are essential for the whole cell catalysis: (i) the transport of the substrates to be hydroxylated, such as Vitamin D₃ in the presence of 7.5% HPCD through the intact outer membrane to the heterologously produced enzyme into the periplasm (=space between outer and inner membrane), (ii) the enzymatic hydroxylation using heterologously produced S25DH with water through the transfer of electrons to acceptors in the periplasm, and (iii) the release (transport back) of the product into the culture medium (Fig. 3). It was found in the context of this invention that hydroxylases, such as, for example S25DH1 or S25DH2 that are heterologously produced in *T*. *aromatica* K172 will transfer electrons onto the cytochrome c as produced by the production strain. Thus, an electric connection is made available from S25DHs to the enzymes of the anaerobic nitrate respiratory chain as produced in the periplasm of the production strain (=denitrification). The inexpensive nitrate is reduced in this process to the inert and volatile N₂, rendering the use of regenerating systems for the electron acceptor superfluous. In typical reactions, 2 mM VitD₃ are converted by whole cells (15 g L⁻¹ dry mass) producing S25DH1 within 40 h to 25-OH-VitD₃ (>85 % conversion); and 2 mM 7-DHC are converted by whole cells producing S25DH2 to 25-OH-7-DHC (>98% conversion). Both conversions are accompanied by a simultaneous reduction of nitrate (10 mM) as added to N₂ (Figure 4). No formation of essential amounts of calcitriol or other by-products was observed (detection limit of mass spectrometric analysis at <0.01%). 2.5 Vitamin D₃ per reduced nitrate are hydroxylated to 25-OH-VitD₃.

### Comparison of whole cell biotransformation of Vitamin D3 to 25-OH-VitD3 with the state of the art.

Table 2 shows a comparison of the biotransformation of VitD3 to 25-OH-VitD₃ according to the present method with several organisms as found in the state of the art (table taken from Tang, D., Liu, W., Huang, L. et al. Efficient biotransformation of vitamin D3 to 25-hydroxyvitamin D3 by a newly isolated Bacillus cereus strain. Appl Microbiol Biotechnol 104, 765-774 (2020). https://doi.org/10.1007/s00253-019-10250-1). It can be seen that the bioconversion is at least 4 times higher than in the most effective comparative strain.

**Table 2 Comparison of whole-cell-based production of 25(OH)VD3 with typical microorganisms (according to Tang et al., 2020). Only the present invention uses water as hydroxylating agent catalysed by a molybdenum-dependent hydroxylase**

| **Strain** | **Bioconversion solvent** | **VD₃ (mg/L)** | **Conversion time (h)** | **25(OH)VD₃ (mg/L)** | **Productivity (mg/L/h)** | **Yield (wt%)** | **Reference** |
|---|---|---|---|---|---|---|---|
| *P. autotrophica* KCTC 1029BP | Ethanol | 600 | 120 | 356 | 2.97 | 59.4 | (Kang et al. 2015b) |
| *P. autotrophica* CGMCC5098 | Ethanol | 1000 | 120 | 639 | 5.33 | 61.3 | (Luo et al. 2017) |
| *P. autotrophica* ID 9302 | Ethanol | 100 | 168 | 10.4 | 0.0619 | 10.4 | (Kang et al. 2006) |
| *A. hyovaginalis* A11-2 | Ethanol | 200 | 100 | 0.118 | 0.0118 | 0.0590 | (Abbas et al. 2011) |
| *A. autotrophica* FERM BP-1573 | Ethanol | 5.00 | ND | ND | ND | ND | (Sasaki et al. 1992) |
| Recombinant *R. erythropolis* | Methylated-β-cyclodextrin | 770 | 2.00 | 0.573 | 0.287 | 74.4 | (Yasutake et al. 2013) |
| *S. lividans* TK23 | Ethanol | 20.5 | 24.0 | 7.96 | 0.332 | 38.9 | (Hayashi et al. 2010) |
| *B. megaterium* MS941 | HP-β-CD | 77.0 | 24.0 | 54.9 | 2.29 | 71.3 | (Abdulmughni et al. 2017) |
| Recombinant S. cerevisiae | Ethanol | 19.2 | 24.0 | 0.881 | 0.0367 | 4.3 | (Yasuda et al. 2013) |
| *B. cereus* zju 4-2 | HP-β-CD Co-solvent | 38.5 2000 | 6.00 32.0 | 27.1 830 | 4.52 25.9 | 70.4 41.5 | (Tang et al., 2020) |
| *Thauera aromatica* K172 | HP-β-CD | 1000 | 40 | 950 | 100 (initial rate) | >85% | Present invention |

### Literature as cited

Abdulmughni A, Józwik IK, Brill E, Hannemann F, Thunnissen AM and Bernhardt R (2017) Biochemical and structural characterization of CYP109A2, a vitamin D3 25-hydroxylase from Bacillus megaterium 284, 3881-3894.
Anders HJ, et al. Taxonomic position of aromatic-degrading denitrifying pseudomonad strains K 172 and KB 740 and their description as new members of the genera Thauera, as Thauera aromatica sp. nov., and Azoarcus, as Azoarcus evansii sp. nov., respectively, members of the beta subclass of the Proteobacteria. Int J Syst Bacteriol. 1995 Apr;45(2):327-33. doi: 10.1099/00207713-45-2-327.
Cashman KD, Seamans KM, Lucey AJ, Stocklin E, Weber P, Kiely M and Hill TR (2012) relative effectiveness of oral 25-hydroxyvitamin D3 and vitamin D3 in raising wintertime serum 25-hydroxyvitamin D in older adults. Am J Clin Nutr 95, 1350-6.
Dermer J and Fuchs G (2012) Molybdoenzyme that catalyzes the anaerobic hydroxylation of a tertiary carbon atom in the side chain of cholesterol. J Biol Chem, 287, 36905-16.
Escribà-Gelonch M, Noël T and Hessel V (2018) Microflow high-p, T intensification of vitamin D3 synthesis using an ultraviolet lamp. Org Process Res Dev 22, 147-155.
Jacoby C, Eipper J, Warnke M, Tiedt O, Mergelsberg M, Stärk HJ, Daus B, Martin-Moldes Z, Zamarro MT, Diaz E, Boll M. (2018) Four Molybdenum-Dependent Steroid C-25 Hydroxylases: Heterologous Overproduction, Role in Steroid Degradation, and Application for 25-Hydroxyvitamin D3 Synthesis. (2018) MBio. 9(3). pii: e00694-18. doi: 10.1128/mBio.00694-18.
Jacoby C, et al. Four Molybdenum-Dependent Steroid C-25 Hydroxylases: Heterologous Overproduction, Role in Steroid Degradation, and Application for 25-Hydroxyvitamin D3 Synthesis. (2018) MBio. 9(3). pii: e00694-18. doi: 10.1128/mBio.00694-18.
Jacoby C, Ferlaino S, Bezold D, Jessen H, Müller M and Boll M (2020) ATP-dependent hydroxylation of an unactivated primary carbon with water. Nat Commun 11, 3906.
Kang DJ, Im JK, Kang JH and Kim KH (2015) Bioconversion of vitamin D to calcifediol by using resting cells of Pseudonocardia sp. Biotechnol Lett 37, 1895-1904.
Lips P (2006) Vitamin D physiology. Prog Biophys Mol Biol 92, 4-8.
Pilz et al. (2018) rationale and plan for vitamin D food fortification: a review and guidance paper. Front Endocrinol 9, 373.
Tang D, Liu W, Huang L, Cheng L and Xu Z (2019) Efficient biotransformation of vitamin D3 to 25-hydroxyvitamin D3 by a newly isolated bacillus cereus strain. Appl Micobiol Cell Physiol 104, 765-774.
Tarlera, S. and Denner EBM (2003) Sterolibacterium denitrificans gen. nov., sp. Nov, a novel cholesterol-oxidizing, denitrifying members of the beta-Proteobacteria. Int J Syst Evol Microbiol 53, 1085-91.
Thayer MT, Nelssen JL, Langemeier AJ, Morton JM, Gonzales JM, Kruger SR, Ou Z, Makowski AJ and Bergstrom JR (2019) The effects of maternal dietary supplementation of cholecalciferol (vitamin D3) and 25-OH-vitamin D3 and progeny performance. Transl. Anim. Sci. 3, 692-708.
Warnke M, Jung T, Dermer J, Hipp K, Jehmlich N, von Bergen M, Ferlaino S, Fries A, Müller M, Boll M (2016) 25-hydroxyvitamin D3 synthesis by enzymatic steroid side-chain hydroxylation with water. Angew Chem Int Ed Engl 55:1881
Wideman, RF, Blankenship J, Pevzner IY and Turner BJ (2015) Efficacy of 25-OH Vitamin D3 prophylatic administration for reducing lameness in broilers grown on wire flooring. Poultry Sciene 94, 1821-7.
Zhu, GD and Okumara WH (1995) synthesis of vitamin D (calciferol), Chem. Rev. 1995, 95, 6, 1877-1952.

## Claims

1. A method for producing 25-hydroxy-vitamin D₃ (25-OH-VitD3), 25-hydroxy-7-dehydrocholesterol (25-OH-7-DHC) and/or analogous alcohols thereof, comprising
a) Providing a recombinant strain of a suitable beta-proteobacterium, preferably *Thauera aromatica* K172, that heterologously overexpresses the genes encoding the three structural subunits of at least one functional water-dependent alkyl-hydroxylase together with at least one chaperone cofactor,
b) Suitably culturing said strain in or on a culture medium with nitrate as terminal electron acceptor for anaerobic respiration in order to obtain resting cells of said strain,
c) Adding vitamin D3 (VitD3) and/or 7-dehydrocholesterol (7-DHC) or other suitable compound comprising a C-H-residue in a tertiary, secondary or primary-allylic or primary-benzylic position to be hydroxylated to said resting cells, and
d) Isolating said 25-OH-VitD₃, 25-OH-7-DHC and/or said analogous alcohol thereof from said culture medium.

2. The method according to claim 1, wherein said water-dependent alkyl-hydroxylase is selected from the group of alkylhydroxlases of the DMSO family of molybdenum-cofactor dependent enzymes and related molybdoenzymes and related water-dependent alkyl-hydroxylases, *S. denitrificans* S25DH1, *S. denitrificans* S25DH2, *S. denitrificans* S25DH4, and *S. denitrificans* S25DH2-4, and wherein said at least one chaperone cofactor is selected from the respective D-subunits thereof and related cofactors thereof, such as *S. denitrificans* S25-DH1 D-subunit.

3. The method according to claim 1 or 2, wherein said at least one water-dependent alkyl-hydroxylase and at least one chaperone cofactor are expressed from a recombinant plasmid, preferably further comprising a selection marker, such as gentamycin resistance.

4. The method according to any one of claims 1 to 3, wherein said conversion uses water as hydroxylating agent.

5. The method according to any one of claims 1 to 4, wherein said culturing is performed in a medium, preferably a synthetic minimal medium, comprising benzoate, ethanol or acetate as electron donor and carbon-source and nitrate as electron acceptor under anaerobic conditions.

6. The method according to any one of claims 1 to 5, wherein said culturing takes place at a high density of cells and/or with resting cells.

7. The method according to any one of claims 1 to 6, wherein said culturing is at about 30°C.

8. The method according to any one of claims 1 to 7, wherein said heterologous overexpression comprises a suitable induction of the recombinant enzymes.

9. The method according to any one of claims 1 to 8, wherein said method does not produce calcitriol and/or does not produce substantial amounts of calcitriol or other undesired by-products.

10. The method according to any one of claims 1 to 9, wherein said method further comprises the addition of an electron acceptor, such as Fe³⁺, for example K₃Fe[CN]₆, DMSO, N₂O, O₂ or other inorganic electron acceptors.

11. The method according to any one of claims 1 to 10, wherein said method further comprises the addition of cyclodextrine, such as 2-hydroxypropyl)-beta-cyclodextrine (HPCD).

12. The method according to any one of claims 1 to 11, wherein said method provides a yield of the conversion of >75%, preferably >80%, more preferably >90%, and mot preferably >95%, such as >99%.

13. The method according to any one of claims 1 to 12, wherein said method is performed as a continuous method, a two-phase method, and/or as a fed batch method.

14. The method according to any one of claims 1 to 13, wherein said method is performed with the recombinant strain of a suitable beta-proteobacterium, preferably *Thauera aromatica* K172, as a biofilm, preferably a biofilm on an electrode material.

15. Use of a recombinant strain of a beta-proteobacterium, preferably *Thauera aromatica* K172, that heterologously overexpresses at least one functional water-dependent alkyl-hydroxylase and at least one chaperone cofactor for the production of 25-hydroxy-vitamin D₃ (25-OH-VitD3), 25-hydroxy-7-dehydrocholesterol (25-OH-7-DHC) and/or analogous alcohols thereof, preferably in a method according to any one of claims 1 to 14.
